# EUROPÄISCHE PATENTANMELDUNG

(11) **EP 3 489 228 A1**
(43) Veröffentlichungstag der Anmeldung: **29.05.2019**
(21) Anmeldenummer: 17203529.7
(22) Anmeldetag: 24.11.2017
(51) Int. Cl.: C07D 401/06, C09B 23/00

(54) **NEUE METHINFARBSTOFFE**

(71) Anmelder: LANXESS Deutschland GmbH, 50569 Köln (DE)
(72) Erfinder: Borst, Hans-Ulrich, 50189 Elsdorf (DE); Linke, Frank, 51069 Köln (DE); MICHAELIS, Stephan, 51519 Odenthal (DE)

(57) **Zusammenfassung**

Die vorliegende Erfindung betrifft neue rote Methinfarbstoffe, Verfahren zu deren Herstellung und deren Verwendung zum Färben von Kunststoffen, insbesondere Vinylpolymeren, wobei rote Färbungen mit verbesserten Farbstärken und verbesserten Farbbrillanzen erhalten werden.

## Beschreibung

Die vorliegende Erfindung betrifft neue Methinfarbstoffe, Verfahren zu deren Herstellung und deren Verwendung zum Färben von Kunststoffen.

Obwohl es auf dem Markt bereits eine Vielzahl roter Farbmittel zur Kunststoffeinfärbung gibt, besteht dennoch Bedarf an neuen Farbmitteln mit verbesserten Eigenschaften. Aus dem Stand der Technik sind bereits Farbstoffe aus verschiedenen Farbstoffklassen bekannt, die für die Färbung von Kunststoffen in roten Farbtönen geeignet sind.

Bekannte lösliche Rotfarbstoffe die zur Kunststoffeinfärbung verwendet werden, sind z.B. Solvent Red 135 (Macrolex® Rot EG) (C.I 564120), Solvent Red 179 (Macrolex® Rot E2G) (C.I 564150), Solvent Red 52 (Macrolex® Rot 5B) (C.I. 68210) sowie Solvent Red 195 (Macrolex® Rot B).

Die koloristischen Eigenschaften dieser, aus dem Stand der Technik bekannten, roten Farbmittel sind jedoch nicht immer ausreichend für die mittlerweile bestehenden technischen Anforderungen bei der Verwendung zur Einfärbung von Kunststoffen. Insbesondere besteht ein Bedarf an roten Farbmitteln für die Massefärbung von Kunststoffen, die gegenüber dem Stand der Technik hinsichtlich ihrer Farbstärke und Brillanz verbessert sind.

Gegenstand der vorliegenden Erfindung sind neue Methinfarbstoffe der Formel (I) 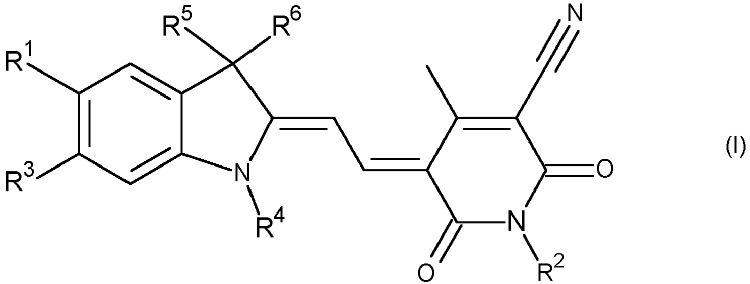 worin
R¹ für Wasserstoff, Halogen, COOH oder COOR⁷ steht,
R² für Wasserstoff oder Alkyl steht,
R³ für Wasserstoff, Halogen, CN, COOH oder COOR⁸ steht,
R⁴ für Alkyl oder Phenyl steht
und
R⁵ und R⁶ unabhängig voneinander jeweils für Alkyl stehen
und
R⁷ und R⁸ unabhängig voneinander jeweils für Alkyl stehen.

Alkyl in den Bedeutungen von R² und R⁴ bis R⁸ bezeichnet einen geradkettigen oder verzweigenten, unsubstituierten oder ein- oder mehrfach gleich oder verschieden substituierten Alkylrest, beispielsweise geradkettiges oder verzweigtes C₁-C₆-Alkyl, vorzugsweise geradkettiges oder verzweigtes C₁-C₄-Alkyl, insbesondere Methyl, Ethyl, n- und iso-Propyl, das jeweils gegebenenfalls ein- oder mehrfach, gleich oder verschieden substituiert sein kann, beispielsweise durch Halogen, wie Chlor, Brom oder Fluor, sowie durch -OH, -CN, -NH₂ oder C₁-C₆-Alkoxy.

Halogen in den Bedeutungen von R¹ und R³ bezeichnet beispielweise Fluor, Chlor oder Brom.

Bevorzugt sind Farbstoffe der Formel (I), worin
R¹ für Wasserstoff, Halogen, COOH oder COOR⁷ steht,
R² für Wasserstoff oder für C₁-C₄-Alkyl, das gegebenenfalls ein- bis dreifach, gleich oder verschieden durch Halogen und/oder Hydroxy substituiert ist, steht,
R³ für Wasserstoff, Halogen, CN, COOH oder COOR⁸ steht,
R⁴ für C₁-C₄- Alkyl oder Phenyl steht,
R⁵ und R⁶ unabhängig voneinander jeweils für C₁-C₄- Alkyl stehen
und
R⁷ und R⁸ unabhängig voneinander jeweils für C₁-C₄-Alkyl stehen.

Besonders bevorzugt sind Farbstoffe der Formel (I), worin
R¹ für Wasserstoff, Fluor, Chlor, COOH oder COOR⁷ steht,
R² für Wasserstoff, Methyl, Ethyl, n- Propyl, iso-Propyl, n-Butyl, iso-Butyl oder tert.-Butyl,
Hydroxymethyl, Hydroxyethyl oder CF₃ steht,
R³ für Wasserstoff, Fluor, Chlor, CN oder COOR⁸ steht,
R⁴ für Methyl oder Phenyl steht,
R⁵ und R⁶ unabhängig voneinander jeweils für Methyl oder Ethyl stehen
und
R⁷ und R⁸ unabhängig voneinander jeweils für Methyl oder Ethyl stehen.

Ganz besonders bevorzugt sind Farbstoffe der Formel (I), worin
R¹ für Wasserstoff, Fluor, Chlor oder COOCH₃ steht,
R² für n-Butyl, iso-Butyl, tert.-Butyl oder Hydroxyethyl steht,
R³ für Wasserstoff, Fluor oder Chlor steht
und
R⁴, R⁵ und R⁶ jeweils für Methyl stehen.

Mit den erfindungsgemäßen Farbstoffen der Formel (I) lassen sich rote Einfärbungen von Kunststoffen erzielen, die sich überraschender Weise gegenüber den mit den bekannten Farbmitteln erzielbaren Einfärbungen sowohl durch eine höhere Farbstärke als auch gleichzeitig durch eine höhere Farbbrillanz auszeichnen.

Mit den erfindungsgemäßen Farbstoffen gelingt es, die bisher realisierten Eigenschaftsprofile bekannter roter Farbstoffe für Kunststoffeinfärbungen deutlich zu übertreffen.

Weiterer Gegenstand der vorliegenden Erfindung ist die Verwendung der erfindungsgemäßen Farbstoffe der Formel (I) zum Massefärben von Kunststoffen. Dabei können die erfindungsgemäßen Farbstoffe einzeln oder in beliebiger Mischung untereinander eingesetzt werden.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits den Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester und Polycarbonate. Ganz besonders bevorzugt sind Vinylpolymere, insbesondere Polystyrol und Polyester, insbesondere Polyethylenterephthalat und Polycarbonat.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u.a..

Geeignete Polyester sind beispielsweise Polyethylenterephthalate, Polybutadienterephthalate, Polycarbonate und Celluloseester.

Die zu färbenden Kunststoffe können einzeln oder in Gemischen untereinander, als plastische Massen oder Schmelzen vorliegen.

Bei ihrem Einsatz zum Massefärben von Kunststoffen werden die erfindungsgemäßen Farbstoffe (I) vorzugsweise in feinverteilter Form zur Anwendung gebracht, wobei Dispergiermittel mitverwendet werden können aber nicht müssen.

Bei ihrem Einsatz zum Massefärben von Kunststoffen können die erfindungsgemäßen Farbstoffe (I) beispielsweise direkt beim Prozess der Kunststoffherstellung nach der erfolgten Polymerisation eingesetzt werden. Dabei wird vorzugsweise mindestens ein erfindungsgemäßer Farbstoff (I) mit dem Kunststoffgranulat trocken vermischt oder vermahlen und dieses Gemisch beispielsweise auf Mischwalzen oder in Schnecken plastifiziert und homogenisiert. Man kann die erfindungsgemäßen Farbstoffe (I) aber auch der schmelzflüssigen Masse zugeben und durch Rühren homogen verteilen. Das derart vorgefärbte Material kann dann wie üblich z.B. durch Verspinnen zu Borsten, Fäden usw. oder durch Extrusion oder im Spritzguss-Verfahren zu Formteilen weiterverarbeitet werden.

Weiterer Gegenstand der vorliegenden Erfindung ist ein Verfahren zum Massefärben von Kunststoffen, dadurch gekennzeichnet, dass mindestens ein Farbstoff der Formel (I) mit mindestens einem geschmolzenen Kunststoff vermischt wird und diese Mischung anschließend homogenisiert wird.

Da die Farbstoffe (I) gegenüber Polymerisationskatalysatoren, insbesondere Peroxiden, beständig sind, ist es auch möglich, die erfindungsgemäßen Farbstoffe (I) den monomeren Ausgangsmaterialien für die Kunststoffherstellung, z. B. von Polymethylmethacrylat (PMMA) zuzusetzen und dann in Gegenwart von Polymerisationskatalysatoren zu polymerisieren. Dazu wird der Farbstoff vorzugsweise in den monomeren Komponenten gelöst oder mit ihnen innig vermischt.

Weiterer Gegenstand der vorliegenden Erfindung ist ein Verfahren zum Massefärben von Polymethylmethacrylat (PMMA) dadurch gekennzeichnet, dass mindestens ein Farbstoff der Formel (I) mit mindestens einem Methacrylsäure-methylestermonomer vermischt oder darin gelöst wird und diese Mischung oder Lösung dann in Gegenwart mindestens eines Polymerisationskatalysators polymerisiert wird.

Die erfindungsgemäßen Farbstoffe der Formel (I) werden vorzugsweise zum Färben der genannten Kunststoffe, insbesondere Polyamid, in Mengen von 0,0001 bis 1 Gew.-%, insbesondere 0,01 bis 0,5 Gew.-%, bezogen auf die Menge an Polymer eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Gegenstand der vorliegenden Erfindung ist weiterhin ein Verfahren zum Massefärben von Kunststoffen, wobei mindestens ein Farbstoff der Formel (I) mit mindestens einem Kunststoff, vorzugsweise in Granulatform, trocken vermischt oder vermahlen wird und dieses Gemisch z.B. auf Mischwalzen oder in Schnecken plastifiziert und homogenisiert wird.

Man kann die erfindungsgemäßen Farbstoffe (I) aber auch der schmelzflüssigen Masse zugeben und durch Rühren homogen verteilen. Ebenfalls ist es möglich, die erfindungsgemäßen Farbstoffe (I) bei der Kunststoffherstellung den monomeren Ausgangskomponenten zuzusetzen und anschließend zu polymerisieren.

Das derart vorgefärbte Material kann dann wie üblich z.B. durch Verspinnen zu Borsten, Fäden usw. oder durch Extrusion oder im Spritzguss-Verfahren zu Formteilen weiterverarbeitet werden.

Nach dem erfindungsgemäßen Verfahren erhält man transparente bzw. gedeckte brillante rote Färbungen, die auch mit sehr guter Hitze- und Lichtbeständigkeiten aufweisen.

Zur Durchführung des erfindungsgemäßen Verfahrens können auch Mischungen der erfindungsgemäßen Farbstoffe der Formel (I) mit anderen Farbstoffen und/oder anorganischen und/oder organischen Pigmenten eingesetzt werden.

Weiterer Gegenstand der vorliegenden Erfindung ist ein Verfahren zur Herstellung der erfindungsgemäßen Farbstoffe der Formel (I).

Die erfindungsgemäßen Farbstoffe der Formel (I) können hergestellt werden, indem man mindestens einen Aldehyd der Formel (II) 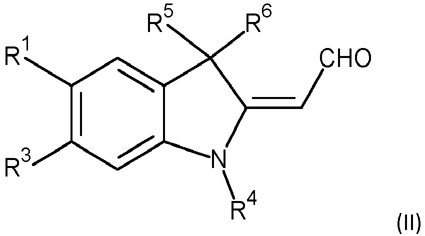 worin
R¹, R³, R⁴, R⁵ und R⁶ die für Formel (I) angegebenen allgemeinen und bevorzugten Bedeutungen haben,
mit mindestens einem Pyridon-Derivat der Formel (III) 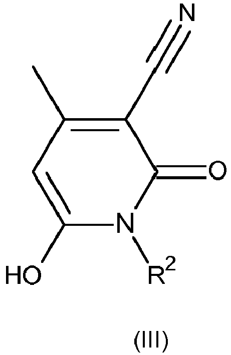 worin
R² die für Formel (I) angegebene allgemeine und bevorzugte Bedeutung hat, umsetzt.

Das Verfahren zur Herstellung der erfindungsgemäßen Farbstoffe (I) durch Umsetzung der Aldehyde der Formel (II) mit den Pyridon-Derivaten der Formel (III) kann in an sich bekannter Art und Weise durchgeführt werden.

Das Verfahren zur Herstellung der erfindungsgemäßen Farbstoffe (I) wird im Allgemeinen bei einer Temperatur im Bereich von -10 bis 180°C, bevorzugt von 0 bis 100°C und besonders bevorzugt von 10 bis 90°C durchgeführt.

Das Verfahren zur Herstellung der erfindungsgemäßen Farbstoffe (I) wird im Allgemeinen bei einem Druck von 900 bis 1100 hPa, vorzugsweise bei Umgebungsdruck durchgeführt. Unter Umgebungsdruck ist ein Luftdruck im Bereich von etwa 925 hPa bis 1070 hPa zu verstehen.

Das Verfahren zur Herstellung der erfindungsgemäßen Farbstoffe (I) kann in Gegenwart von mindestens einem Lösungsmittel durchgeführt werden. Geeignete Lösungsmitte sind beispielsweise solche aus der Reihe der Alkohole und Formamide. Vorzugsweise wird das Verfahren zur Herstellung der erfindungsgemäßen Farbstoffe (I) in Gegenwart von mindestens einem Alkohol aus der Reihe Methanol, Ethanol, Propanol, und/oder mindestens einem Formamid aus der Reihe Dimethylformamid und Diethylformamid, besonders bevorzugt in Gegenwart von Methanol und/oder Dimethylformamid durchgeführt.

Das Verfahren zur Herstellung der erfindungsgemäßen Farbstoffe (I) wird in Gegenwart von mindestens einer Base durchgeführt. Geeignete Basen sind beispielsweise Alkalihydroxide und Alkalialkoholate. Bevorzugt ist die Verwendung von Lithiumhydroxid, Natriumhydroxid, Kaliumhydroxid und/oder Kalium-tert.-butylat, besonders bevorzugt von Natriumhydroxid und/oder Kalium-tert.-butylat.

Im Allgemeinen wird das Verfahren zur Herstellung der erfindungsgemäßen Farbstoffe (I) so durchgeführt, dass zunächst der Aldehyd (II) vorlegt und das Pyridon-Derivat (III) hinzugefügt wird und nach erfolgter Umsetzung die Verbindungen der Formel (I) isoliert wird. Die Isolierung kann durch übliche Verfahren, vorzugsweise durch Filtration, erfolgen. Das erhaltene Reaktionsprodukt kann gegebenenfalls durch weitere Verfahrensschritte wie Waschen und Trocknen aufgearbeitet werden.

Zur Durchführung des Verfahrens wird im Allgemeinen pro Mol an Aldehyd (II) 0,8 bis 1,5 Mol an Pyridon-Derivat (III) eingesetzt. Bevorzugt wird pro Mol an Aldehyd (II) 0,9 bis 1,1 Mol an Pyridon-Derivat (III) eingesetzt und besonders bevorzugt wird pro Mol an Aldehyd (II) 1 Mol an Pyridon-Derivat (III) eingesetzt.

Pyridon-Derivate der Formel (III) sind bekannt und können beispielsweise als Handelsprodukte der Firma Alfa Acer bezogen werden.

Die Aldehyde der Formel (II) sind ebenfalls bekannt und können beispielsweise, in dem Fachmann bekannter Art und Weise, in einer zweistufigen Synthese hergestellt werden. Dabei wird in einer ersten Stufe a) mindestens ein Indol-Derivat der Formel (IV) 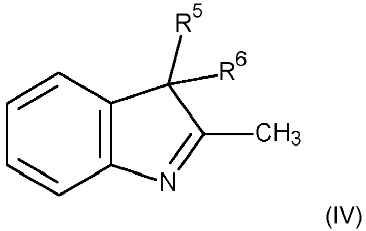 worin
R⁵ und R⁶ die für Formel (I) angegebenen allgemeinen und bevorzugten Bedeutungen haben,
mit mindestens einem Alkylierungsreagenz umgesetzt, und anschließend in einer zweiten Stufe b) das Zwischenprodukt der ersten Stufe mit mindestens einem Formylierungsreagenz umgesetzt.

Umsetzungen der in Stufe b) beschrieben Art sind ist in der Literatur unter dem Namen Vilsmeier-Reaktion bekannt.

Im Allgemeinen wird die Umsetzung in Stufe a) so durchgeführt, dass man das Indol-Derivat der allgemeinen Formel (IV) vorlegt und gegebenenfalls in Gegenwart eines Lösungsmittels das Alkylierungsmittel hinzufügt.

Die erste Stufe a) der Umsetzung wird im Allgemeinen bei einer Temperatur im Bereich zwischen 10 und 80°C bevorzugt von 20 bis 70°C und besonders bevorzugt von 30 bis 60°C durchgeführt.

Die Umsetzung in Stufe a) wird im Allgemeinen bei einem Druck von 900 bis 1100 hPa, vorzugsweise bei Umgebungsdruck durchgeführt. Unter Umgebungsdruck ist ein Luftdruck im Bereich von etwa 925 hPa bis 1070 hPa zu verstehen.

Die Umsetzung in Stufe a) kann in Gegenwart von mindestens einem Lösungsmittel durchgeführt werden. Geeignete Lösungsmitte sind beispielsweise solche aus der Reihe der Alkohole und Wasser. Vorzugsweise wird die Umsetzung in Stufe a) in Gegenwart von Wasser als Lösungsmittel durchgeführt.

Als Alkylierungsreagenz geeignet sind prinzipiell alle bekannten Alkylierungsreagenzien (siehe z. B. K. Schwetlick, Organikum, VEB Deutscher Verlag der Wissenschaften, Berlin, 15. Auflage 1977, Seite 260, 253, 674), wie beispielsweise Dimethylsulfat, Methyliodid oder Diazomethan. Bevorzugt ist die Verwendung von Dimethylsulfat.

Im allgemeinen wird pro Mol an Indol-Derivat mindestens ein Mol an Alkylierungsreagenz eingesetzt. Abhängig von der Struktur des Indol-Derivats kommen, entsprechend der vorgegebenen Stöchiometrie, auch höhere Molmengen zum Einsatz. Vorzugsweise wird pro Mol an Indol-Derivat (IV) 0,9 bis 1,1 Mol, besonders bevorzugt 1 Mol an Alkylierungsreagenz eingesetzt.

Das in Stufe a) hergestellte Zwischenprodukt kann durch übliche Verfahren, beispielsweise durch Filtration isoliert werden. Vorzugsweise wird das in Stufe a) hergestellte Zwischenprodukt ohne Isolierung direkt weiter in der anschließenden Stufe b) umgesetzt.

Im Allgemeinen wird die Umsetzung in Stufe b) so durchgeführt, dass man die alkylierte Verbindung aus der ersten Stufe a) in Form der erhaltenen Reaktionslösung vorlegt und das Formylierungsreagenz, gegebenenfalls in Gegenwart mindestens eines Lösungsmittels, hinzufügt und anschließend den so hergestellten Aldehyd der Formel (II), gegebenenfalls durch Zugabe einer geeigneten Menge eines geeigneten Fällungsmittels ausfällt, und anschließen den Aldehyd der Formel (II) durch übliche Verfahren, beispielsweise durch Filtration, isoliert.

Die Umsetzung in Stufe b) wird im Allgemeinen bei einer Temperatur im Bereich zwischen 10 und 80°C bevorzugt von 20 bis 70°C und besonders bevorzugt von 30 bis 60°C durchgeführt.

Die Umsetzung in Stufe b) wird im Allgemeinen bei einem Druck 900 bis 1100 hPa, vorzugsweise bei Umgebungsdruck durchgeführt. Unter Umgebungsdruck ist ein Luftdruck im Bereich von etwa 925 hPa bis 1070 hPa zu verstehen.

Die Umsetzung in Stufe b) kann in Gegenwart von mindestens einem Lösungsmittel durchgeführt werden. Geeignete Lösungsmitte sind beispielsweise Formamide. Bevorzugt sind Dimethylformamid, und Diethylformamid, besonders bevorzugt ist die Verwendung von Dimethylformamid. Bei Verwendung von Dimethylformamid ist es insbesondere bevorzugt dieses im Überschusses einzusetzen, wobei das Dimethylformamid dann zugleich als Formylierungsreagenz und als Lösungsmittel dient.

Als Formylierungsreagenz wird in Stufe b) im Allgemeinen eine Mischung aus mindestens einem Formamid und mindestens einem Phosphorsäurechlorid eingesetzt.
Bevorzugte Formamide sind Dimethylformamid, Diethylformamid und Dibutylformamid. Bevorzugtes Phosphorsäurechlorid ist Phosphoroxychlorid.

Besonders bevorzugt wird als Formylierungsreagenz eine Mischung aus Dimethylformamid und Phosphoroxychlorid eingesetzt.

Im Allgemeinen wird pro Mol an alkylierter Verbindung aus Stufe 1 mindestens ein Mol an Formylierungsreagenz eingesetzt, bevorzugt 1,1 bis 1,5 Mol und besonders bevorzugt 1,1 bis 1 Mol.

Geeignete Fällungsmittel sind beispielsweise Alkohole wie Methanol und/oder Ethanol.

Bevorzugt wird als Fällungsmittel Methanol und/oder Ethanol, insbesondere Methanol eingesetzt.

Die Indol-Derivate der Formel (IV) sind dem Fachmann bekannt. Sie können in an sich bekannter Art und Weise in einer zweistufigen Synthese hergestellt werden durch Umsetzung eines Anilin-Derivats der Formel (V) 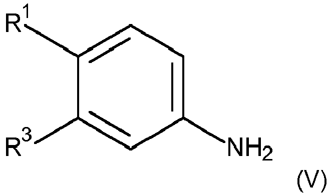 worin
R¹ und R³ die für Formel (I) angegebene allgemeine und bevorzugte Bedeutung haben,
mit einem Diazotierungsreagenz und anschließender Umsetzung unter Ringschluss mit einem Keton der Formel (VI) 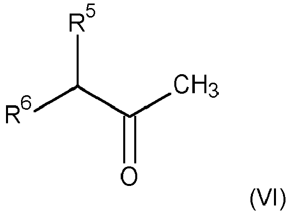 worin
R⁵ und R⁶ die für Formel (I) angegebene allgemeine und bevorzugte Bedeutung haben. Die Diazotierungsreaktion wird im allgemeinen durchgeführt, indem man das Anilin-Derivat vorlegt und das Diazotierungsreagenz bei einer Temperatur im Bereich zwischen 0 bis 10°C , bei Normaldruck in einem wässerigen Medium hinzufügt.

Als Diazotierungsreagenz kommt prinzipiell jedes geeignete Diazotierungsreagenz in Frage. Bevorzugt ist die Verwendung einer wässerigen Natriumnitrit-Lösung.

Im Allgemeinen wird das Diazotierungsreagenz in einer Menge von mindestens zwei Mol bezogen auf das Anilin-Derivat (V) eingesetzt.

Die Ringschluss-Reaktion mit dem Keton der Formel (VI) wird in an sich bekannter Art und Weise einer Eintopf-Reaktion durchgeführt, indem man das Diazoniumsalz des Anilin-Derivats (V) zum Hydrazon reduziert und das Hydrazon mit dem Keton der allgemeinen Formel (VI) umsetzt, bevorzugt bei einer Temperatur im Bereich von 40 bis 100°C, bevorzugt in wässriger Lösung, und anschließend das Indol-Derivat der Formel (IV) durch übliche Verfahren, vorzugsweise Filtration, isoliert und wäscht.

Die Anilin-Derivate der Formel (V) sowie die Ketone der Formel (VI) sind bekannt und können als Handelsprodukte bezogen werden, beispielsweise von den Firmen Alfa Acer oder Sigma-Aldrich.

Die Erfindung wird erläutert, jedoch nicht beschränkt durch die folgenden Beispiele, in denen die Teile sich auf das Gewicht beziehen und Prozentangaben Gewichtsprozente (Gew.-%) bedeuten.

### Beispiel 1

### Herstellung der erfindungsgemäßen Verbindung der Formel (I)

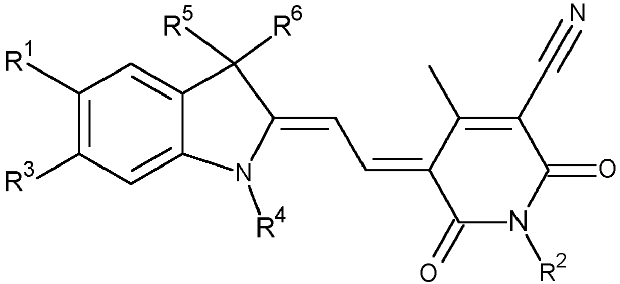

mit R¹ = COOCH₃; R² = C₄H₉; R³ = H und R⁴, R⁵ und R⁶ = CH₃

In 160 ml Essigsäureanhydrid wurden 25,9 g (0,1 Mol) Aldehyd der Formel (II) mit R¹ = COOCH₃; R³ = H und R⁴, R⁵ und R⁶ = CH₃ sowie 20,6 g (0,1 Mol) N-Butyl-6-hydroxy-3-cyano-4-methyl-2-pyridon sowie 5 g Ammoniumchlorid eingetragen. Anschließend wurde die Reaktionsmischung auf eine Temperatur von 105 °C erwärmt und für ca. 6 Stunden gerührt. Danach wurde auf 25°C abgekühlt und mit 200 ml Methanol versetzt und das Reaktionsprodukt auf einer Nutsche isoliert. Der Filterkuchen wurde mit ca. 600 ml Methanol und ca. 4000 ml Wasser mit einer Temperatur von 90°C gewaschen. Das gewaschene Produkt wurde im Vakuumtrockenschrank bei einer Temperatur von 80°C und einem Druck von 200 hPa getrocknet.
Ausbeute: 38,2 g (entspricht 85% der Theorie), Schmelzpunkt 261°C

### Beispiele 2 bis 4

Herstellung von erfindungsgemäßen Verbindungen der Formel (I) worin die Substituenten R¹ bis R⁶ die in Tabelle 1 aufgeführten Bedeutungen haben.

**Tabelle 1**

| Beispiel | R¹ | R² | R³ | R⁴ | R⁵ | R⁶ |
|---|---|---|---|---|---|---|
| 2 | H | C₄H₉ | H | CH₃ | CH₃ | CH₃ |
| 3 | Cl | C₄H₉ | H | CH₃ | CH₃ | CH₃ |
| 4 | H | C₄H₉ | F | CH₃ | CH₃ | CH₃ |

Die Herstellung und Aufarbeitung der Verbindungen der Beispiele 2 bis 4 erfolgte jeweils analog zu Beispiel 1, jedoch mit folgenden Abweichungen:

### Beispiel 2

Es wurden 90 ml Essigsäure und 160 ml Essigsäureanhydrid vorgelegt. Anstelle des in Beispiel 1 verwendeten Aldehyds wurden 20,1 g (0,1 Mol) Aldehyd der Formel (II) mit R¹ und R³ = H und R⁴, R⁵ und R⁶ = CH₃ sowie 4 g Ammoniumchlorid eingesetzt. Nach dem Abkühlen auf 25°C wurde mit 170 ml Methanol versetzt, das Reaktionsprodukt auf einer Nutsche isoliert und der Filterkuchen mit 100 ml Methanol und ca. 400 ml Wasser mit einer Temperatur von 90°C gewaschen
Ausbeute: 35,2 g (entspricht 90 % der Theorie), Schmelzpunkt 254°C

### Beispiel 3

Es wurden 190 ml Essigsäure vorgelegt. Anstelle des in Beispiel 1 verwendeten Aldehyds wurden 23,6 g (0,1 Mol) Aldehyd der Formel (II) mit R¹ = Cl; R³ = H und R⁴, R⁵ und R⁶ = CH₃ sowie 6 g Ammoniumchlorid eingesetzt. Nach dem Abkühlen auf 25°C wurde mit 250 ml Methanol versetzt, das Reaktionsprodukt auf einer Nutsche isoliert und der Filterkuchen mit 150 ml Methanol und ca. 600 ml Wasser mit einer Temperatur von 90°C gewaschen
Ausbeute: 33,2 g (entspricht 78 % der Theorie), Schmelzpunkt 273°C

### Beispiel 4

Es wurden 190 ml Essigsäure vorgelegt. Anstelle des in Beispiel 1 verwendeten Aldehyds wurden 21,9 g (0,1 Mol) Aldehyd der Formel (II) mit R¹ = H; R³ = F und R⁴, R⁵ und R⁶ = CH₃ sowie 2,5 g Ammoniumchlorid eingesetzt. Nach dem Abkühlen auf 25°C wurde mit 200 ml Methanol versetzt, das Reaktionsprodukt auf einer Nutsche isoliert und der Filterkuchen mit 150 ml Methanol und ca. 600 ml Wasser mit einer Temperatur von 90°C gewaschen
Ausbeute: 33,8 g (entspricht 86 % der Theorie), Schmelzpunkt 253°C

### Herstellung der Vorstufen

### Beispiel 5

Herstellung eines Aldehyds der Formel (II) 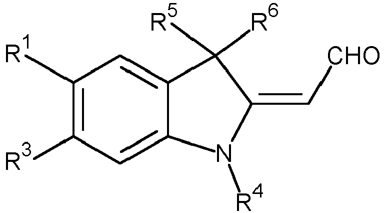 mit R¹ = COOCH₃; R³ = H und R⁴, R⁵ und R⁶ = CH₃

### a) Diazotierung:

In 270 g 30%ige Salzsäure wurden 139,9 g p-Aminobenzoesäure eingetragen und durch Kühlung von außen auf 0°C abgekühlt. Anschließend wurde mit 174 g einer 40%igen wässrigen Natriumnitrit Lösung versetzt. Es wurde für 30 Minuten gerührt und dann mit ca. 0,5 g Amidosulfonsäure der Nirit-Überschuss entfernt.

### b) Herstellung des Hydrazons und Ringschluss:

Eine Mischung aus 250 g Wasser und 660 g Natriumhydrogensulfit, in Form einer 39%igen wässrigen Lösung, wurde mit 80 g einer 40%igen wässrigen Natriumhydroxid-Lösung auf einen pH-Wert von 6,5 eingestellt. Im Laufe von ca. 30 Minuten wurde die in Schritt a) hergestellte Diazotierungslösung zugegeben, wobei durch Zugabe von 100 g einer 40%igen wässrigen Natriumhydroxid-Lösung der pH-Wert bei ca. 6,5 gehalten wurde. Anschließend wurde die Reaktionsmischung bei einer Temperatur von 40°C für ca. 1 Stunde gerührt. Danach wurden 560 g 96%ige Schwefelsäure und anschließend 86,1 g Methylisopropylketon zugetropft. Die Reaktionsmischung wurde auf 70°C erwärmt und für ca. 4 Stunden gerührt. Anschließend wurde die Reaktionsmischung auf 80°C erwärmt und nochmals für ca. 4 Stunden gerührt. Danach wurde die Reaktionsmischung auf 25°C abgekühlt und mit ca. 800 g einer 40%igen wässrigen Natriumhydroxid-Lösung auf einen pH-Wert von 6,5 eingestellt. Die Reaktionsmischung wurde für 30 Minuten gerührt und anschließend das Reaktionsprodukt auf einer Nutsche isoliert und mit 2 Liter Wasser gewaschen.

### c) Herstellung des Aldehyds:

Der wasserfeuchte Presskuchen des Ringschlussproduktes aus Stufe b) wurde in 1200 g Wasser eingetragen. Anschließend wurde mit ca. 70 g einer 40%igen wässrigen Natriumhydroxid-Lösung ein pH-Wert von 10 eingestellt. Im Laufe von 1 Stunde wurden 325 g Dimethylsulfat zugetropft und dabei durch Zugabe von 200 g einer 40%igen wässrigen Natriumhydroxid-Lösung der pH-Wert bei ca. 8,5 gehalten. Die Reaktionsmischung wurde auf 40°C erwärmt und für ca. 5 Stunden gerührt. Anschließend wurde die Reaktionsmischung auf 60°C erwärmt und nochmals für 1 Stunde gerührt. Die Reaktionsmischung wurde dann stehen gelassen wobei innerhalb von 1 Stunde eine Phasentrennung erfolgte. Dann wurde die wässrige Phase abgetrennt. Im Vakuum bei 80°C und 20 hPa wurde restliches Wasser aus der organischen Phase entfernt. Danach wurden zu der organischen Phase 310 g Dimethylformamid zugetropft. Anschließend wurden bei 40°C 263 g Phosphoroxychlorid im Laufe von 3 Stunden zugegeben und die Reaktionsmischung für 5 Stunden gerührt. Anschließend wurde auf 20°C abgekühlt und mit 160 g Methanol versetzt. Dann wurde mit ca. 200 g einer 40%igen wässrigen Natriumhydroxid-Lösung ein pH-Wert von 11 eingestellt. Anschließend wurde die Reaktionsmischung für 60 Minuten gerührt und dann das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 hPa getrocknet.
Ausbeute: 176,3 g (entspricht 68% der Theorie)

### Beispiel 6

Herstellung eines Aldehyds der Formel (II) mit R¹ = Cl, R³ = H und R⁴, R⁵ und R⁶ = CH₃

### a) Diazotierung:

Die Herstellung der Diazotierungslösung erfolgte wie in Beispiel 5 a) angegeben, jedoch wurden 268 g 30%-ige Salzsäure eingesetzt und anstelle von p-Aminobenzoesäure 127,6 g 4-Chloranilin verwendet.

### b) Herstellung des Hydrazons und Ringschluss:

Die Herstellung des Hydrazons erfolgte analog zu Beispiel 5 a), es wurde jedoch die Diazotierungslösung aus Beispiel 6 a) eingesetzt.

### c) Herstellung des Aldehyds:

Der wasserfeuchte Presskuchen des Ringschlussproduktes aus Stufe b) wurde in 1200 g Wasser eingetragen. Anschließend wurde mit ca. 5 g einer 40%igen wässrigen Natriumhydroxid-Lösung ein pH-Wert von 10 eingestellt. Im Laufe von 1 Stunde wurden 153 g Dimethylsulfat zugetropft und dabei durch Zugabe von 90 g einer 40%igen wässrigen Natriumhydroxid-Lösung der pH-Wert bei ca. 8,5 gehalten. Die Reaktionsmischung wurde auf 40°C erwärmt und für ca. 5 Stunden gerührt. Anschließend wurde die Reaktionsmischung auf 60°C erwärmt und nochmals für 1 Stunde gerührt. Die Reaktionsmischung wurde dann stehen gelassen wobei innerhalb von 1 Stunde eine Phasentrennung erfolgte. Dann wurde die wässrige Phase abgetrennt. Im Vakuum bei 80°C und 20 hPa wurde restliches Wasser aus der organischen Phase entfernt. Danach wurden zu der organischen Phase 275 g Dimethylformamid zugetropft. Anschließend wurden bei 40°C 116 g Phosphoroxychlorid im Laufe von 3 Stunden zugegeben und die Reaktionsmischung für 5 Stunden gerührt. Anschließend wurde auf 20°C abgekühlt und mit 160 g Methanol versetzt. Dann wurde mit ca. 180 g einer 40%igen wässrigen Natriumhydroxid-Lösung ein pH-Wert von 11 eingestellt. Anschließend wurde die Reaktionsmischung für 60 Minuten gerührt und dann das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 hPa getrocknet.
Ausbeute: 141,4 g (entspricht 60% der Theorie)

### Beispiel 7

Herstellung eines Aldehyds der Formel (II) mit R¹ = H, R³ = F und R⁴, R⁵ und R⁶ = CH₃

### a) Diazotierung:

Die Herstellung der Diazotierung erfolgte wie in Beispiel 5 a) angegeben, jedoch wurden 375 g 30%ige Salzsäure eingesetzt und anstelle von p-Aminobenzoesäure wurden 155,5 g 3-Fluoranilin verwendet.

### b) Herstellung des Hydrazons und Ringschluss:

Eine Mischung aus 250 g Wasser und 918 g Natriumhydrogensulfit, in Form einer 39%igen wässrigen Lösung, wurde mit 120 g einer 40%igen wässrigen Natriumhydroxid-Lösung auf einen pH-Wert von 6,5 eingestellt. Im Laufe von ca. 30 Minuten wurde die in Schritt a) hergestellte Diazotierungslösung zugegeben, wobei durch Zugabe von 140 g einer 40%igen wässrigen Natriumhydroxid-Lösung der pH-Wert bei ca. 6,5 gehalten wurde. Anschließend wurde die Reaktionsmischung bei einer Temperatur von 40°C für ca. 1 Stunde gerührt. Danach wurden 776 g 96%ige Schwefelsäure und anschließend 120,4 g Methylisopropylketon zugetropft. Die Reaktionsmischung wurde auf 70°C erwärmt und für ca. 4 Stunden gerührt. Anschließend wurde die Reaktionsmischung auf 80°C erwärmt und nochmals für ca. 4 Stunden gerührt. Danach wurde die Reaktionsmischung auf 25°C abgekühlt und mit ca. 1150 g einer 40%igen wässrigen Natriumhydroxid-Lösung auf einen pH-Wert von 6,5 eingestellt. Die Reaktionsmischung wurde für 30 Minuten gerührt und anschließend das Reaktionsprodukt auf einer Nutsche isoliert und mit 2 Liter Wasser gewaschen.

### c) Herstellung des Aldehyds:

Der wasserfeuchte Presskuchen des Ringschlussproduktes aus Stufe b) wurde in 1200 g Wasser eingetragen. Anschließend wurde mit ca. 10 g einer 40%igen wässrigen Natriumhydroxid-Lösung ein pH-Wert von 10 eingestellt. Im Laufe von 1 Stunde wurden 194 g Dimethylsulfat zugetropft und dabei durch Zugabe von 120 g einer 40%igen wässrigen Natriumhydroxid-Lösung der pH-Wert bei ca. 8,5 gehalten. Die Reaktionsmischung wurde auf 40°C erwärmt und für ca. 5 Stunden gerührt. Anschließend wurde die Reaktionsmischung auf 60°C erwärmt und nochmals für 1 Stunde gerührt. Die Reaktionsmischung wurde dann stehen gelassen wobei innerhalb von 1 Stunde eine Phasentrennung erfolgte. Dann wurde die wässrige Phase abgetrennt. Im Vakuum bei 80°C und 20 hPa wurde restliches Wasser aus der organischen Phase entfernt. Danach wurden zu der organischen Phase 350 g Dimethylformamid zugetropft. Anschließend wurden bei 40°C 147 g Phosphoroxychlorid im Laufe von 3 Std. zugegeben und die Reaktionsmischung für 5 Stunden gerührt. Anschließend wurde auf 20°C abgekühlt und mit 160 g Methanol versetzt. Dann wurde mit ca. 200 g einer 40%igen wässrigen Natriumhydroxid-Lösung ein pH-Wert von 11 eingestellt. Anschließend wurde die Reaktionsmischung für 60 Minuten gerührt und dann das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 hPa getrocknet.
Ausbeute: 169,1 g (entspricht 55% der Theorie)

### Liste der zugekauften Einsatzstoffe:

| Bezeichnung: | Molgewicht | Cas. Nr. | Gehalt | Hersteller |
|---|---|---|---|---|
| p-Aminobenzoesäure | 137,2 | 150-13-0 | 98 | Sigma-Aldrich |
| | | | | |
| Methylisopropylketon | 86,1 | 563-80-4 | 99 | Sigma-Aldrich |
| Isopropyl methyl ketone | | | | |
| 4- Chloranilin | 127,6 | 106-47-8 | 98 | Sigma-Aldrich |
| 3-Fluoranilin | 111,1 | 372-19-0 | 99 | Alfa Acer |
| 2-(1,3,3-Trimethylindolin-2-yliden)-acetaldehyd | 201,3 | 84-83-3 | 97 | Sigma-Aldrich |

### Spektroskopische Messungen

Die Ergebnisse der UV/VIS Messungen und Extinktionswerte für die erfindungsgemäßen Verbindungen der Beispiele 1 bis 4 und die der nicht erfindungsgemäßen Vergleichsverbindungen sind in Tabelle 2 aufgeführt.

Die UV/VIS-Absorptionsspektren der erfindungsgemäßen und nicht erfindungsgemäßen Verbindungen wurden alle im Lösungsmittel 1-Methoxy-2-propylacetat (CAS-Nr. 108-65-6) gemessen.

**Tabelle 2**

| Beispiel/ Vergleich | Erfindungsgemäß | Absorptionsmaximum UV/VIS-Spektrum¹⁾ | E 1/1-Wert²⁾ |
|---|---|---|---|
| Beispiel 1 | ja | 526 nm | 2672 |
| Beispiel 2 | ja | 524 nm | 2890 |
| Beispiel 3 | ja | 521 nm | 3173 |
| Beispiel 4 | ja | 520 nm | 2874 |
| Macrolex® Rot EG | nein | 496 nm | 160 |
| Macrolex® Rot E2G | nein | 473 nm | 290 |
| Macrolex® Rot B | nein | 528 nm | 900 |
| Macrolex® Rot 5 B | nein | 539 nm | 350 |

| | | | |
|---|---|---|---|
| ²⁾ Der angegebene E1/1-Wert ist ein fiktiver Extinktionswert. Gemessen wird zunächst die Extinktion einer Lösung der jeweiligen Probe in 1-Methoxy-2-propylacetat in einer Küvette der Schichtdicke von 1 cm, wobei die Konzentration der Lösung so gewählt wird, dass der beobachtete Extinktionswert im Absorptionsmaximum etwa 1 beträgt. Der ermittelte Wert wird dann auf eine Konzentration von 1 Gewichtsprozent umgerechnet wodurch sich der E 1/1-Wert ergibt. | | | |

Farbmittel, die sowohl hohe Farbstärke als auch hohe Farbbrillanz aufweisen, sind dadurch charakterisiert, dass sie Absorptionsbanden im sichtbaren Spektralbereich mit geringen Halbwertsbreiten aufweisen und gleichzeitig hohe Extinktionskoeffizienten an der Wellenlänge des Absorptionsmaximums vorliegen.

Die berechneten Halbwertsbreiten (HWB) und molaren Extinktionskoeffizienten im Absorptionsmaximum (ε max) für die erfindungsgemäßen und nicht erfindungsgemäßen Verbindungen sind in Tabelle 3 aufgeführt.

**Tabelle 3**

| Beispiel/ Vergleich | Erfindungsgemäß | HWB* [nm] | ε max [l/mol*cm] |
|---|---|---|---|
| 1 | ja | 47 | 124000 |
| 2 | ja | 46 | 127000 |
| 3 | ja | 40 | 128000 |
| 4 | ja | 46 | 121000 |
| Macrolex® Rot EG | nein | 126 | 6700 |
| Macrolex® Rot E2G | nein | 117 | 9600 |
| Macrolex® Rot B | nein | 84 | 38000 |

*Die Halbwertsbreite gibt dabei die Breite der Verteilung bei halbem Maximalwert der Verteilung an. Man unterscheidet in der Regel die volle Halbwertsbreite (engl.: full width at half maximum, FWHM) und die halbe Halbwertsbreite (engl.: half width at half maximum, HWHM). Die in Tabelle 3 angegebenen Halbwertsbreiten entsprechen den vollen Halbwertsbreiten (FWHM).

### Bestimmung der Farbstärke und Brillanz

Zur Bestimmung der Farbstärke und Brillanz der erfindungsgemäßen Proben der Beispiele 1 bis 4 sowie des nicht erfindungsgemäßen Vergleichsbeispiels wurde jede Probe einer koloristischen Messung gemäß der unten angegebenen Vorschrift unterzogen.

500 g mit 2 Gew.-% Titandioxid eingefärbtes Polystyrol wurde mit 0,10 Gew.-% Farbstoffpulver durch Aufschütteln in einem geschlossenen Kunststoffbeutel vermischt. Aus dem homogenen Granulat-Farbstoff-Gemisch wurden Musterplättchen von 4 cm x 6 cm x 0,2 cm auf einer Spritzgießmaschine bei 240 °C Massetemperatur, 20 bar Staudruck und 60 °C Formtemperatur hergestellt.

Nach frühestens zehn Spritzzyklen wurden Musterplättchen für die Farbmessung entnommen und mindestens 1 Stunde bei Raumtemperatur ruhen gelassen

Mit einem d/8° Spektralphotometer wurden von den Musterplättchen Remissionsmessungen durchgeführt. Die Bestimmung der Farbstärke und der Restfarbabstände erfolgte nach DIN 55986, die Bestimmung der Brillanz nach DIN EN ISO 11664-4.

Die Ergebnisse der Farbbrillanz- und Farbstärke-Messungen für die erfindungsgemäßen und nicht erfindungsgemäßen Verbindungen sind in Tabelle 4 aufgeführt.

**Tabelle 4**

| Beispiel/ Vergleich | Erfindungsgemäß | Farbstärke | Brillanz dC |
|---|---|---|---|
| 1 | ja | 131 | 8,8 |
| 2 | ja | 135 | 8,9 |
| 3 | ja | 141 | 10,7 |
| 4 | ja | 132 | 8,8 |
| Macrolex® Rot B | nein | 100 | 0 |

## Patentansprüche

1. Farbstoff der Formel (I) 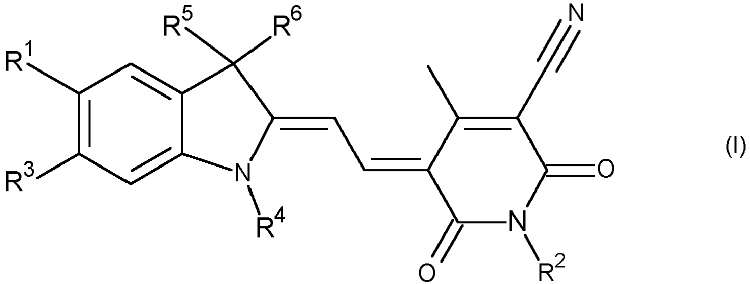
R¹ für Wasserstoff, Halogen, COOH oder COOR⁷ steht,
R² für Wasserstoff, CF₃ oder Alkyl steht,
R³ für Wasserstoff, Halogen, COOH, COOR⁸ oder CN steht,
R⁴ für Alkyl oder Phenyl steht
und
R⁵ und R⁶ unabhängig voneinander jeweils für Alkyl stehen
und
R⁷ und R⁸ unabhängig voneinander jeweils für Alkyl stehen.

2. Farbstoff gemäß Anspruch 1, **dadurch gekennzeichnet, dass** in Formel (I)
R¹ für Wasserstoff, Halogen, COOH oder COOR⁷ steht,
R² für Wasserstoff oder für C₁-C₄-Alkyl, das gegebenenfalls ein- bis dreifach, gleich oder verschieden durch Halogen und/oder Hydroxy substituiert ist, steht,
R³ für Wasserstoff, Halogen, CN, COOH oder COOR⁸ steht,
R⁴ für C₁-C₄- Alkyl oder Phenyl steht,
R⁵ und R⁶ unabhängig voneinander jeweils für C₁-C₄- Alkyl stehen
und
R⁷ und R⁸ unabhängig voneinander jeweils für C₁-C₄- Alkyl stehen.

3. Farbstoff gemäß Anspruch 1 oder 2, **dadurch gekennzeichnet, dass** in Formel (I) R¹ für Wasserstoff, Fluor, Chlor, COOH oder COOR⁷ steht,
R² für Wasserstoff, Methyl, Ethyl, n- Propyl, iso-Propyl, n-Butyl, iso-Butyl oder tert.-Butyl, Hydroxymethyl, Hydroxyethyl oder CF₃, steht,
R³ für Wasserstoff, Fluor, Chlor, CN oder COOR⁸ steht,
R⁴ für Methyl oder Phenyl steht,
R⁵ und R⁶ unabhängig voneinander jeweils für Methyl oder Ethyl stehen
und
R⁷ und R⁸ unabhängig voneinander jeweils für Methyl oder Ethyl stehen.

4. Farbstoff gemäß wenigstens einem der Ansprüche 1 bis 3, **dadurch gekennzeichnet, dass** in Formel (I)
R¹ für Wasserstoff, Fluor, Chlor oder COOCH₃ steht,
R² für n-Butyl, iso-Butyl, tert.-Butyl oder Hydroxyethyl steht,
R³ für Wasserstoff, Fluor oder Chlor steht
und
R⁴, R⁵ und R⁶ jeweils für Methyl stehen.

5. Verwendung von mindestens einem Farbstoff gemäß wenigstens einem der Ansprüche 1 bis 4 zum Massefärben von Kunststoffen.

6. Verwendung gemäß Anspruch 5, **dadurch gekennzeichnet, dass** es sich um mindestens einen Kunststoff aus der Reihe der Vinylpolymere, Polyester, und Polycarbonate handelt.

7. Verwendung gemäß wenigstens einem der Ansprüche 5 oder 6, **dadurch gekennzeichnet, dass** es sich bei dem Kunststoff um Polystyrol, Polymethylmethacrylat (PMMA), Polyethylenterephthalat oder Polycarbonat, handelt.

8. Verwendung gemäß wenigstens einem der Ansprüche 5 bis 7, **dadurch gekennzeichnet, dass** der Farbstoff in einer Menge von 0,0001 bis 1 Gewichtsprozent, insbesondere 0,01 bis 0,5 Gewichtsprozent, bezogen auf die Menge an Kunststoff eingesetzt wird.

9. Verfahren zum Massefärben von Kunststoffen, **dadurch gekennzeichnet, dass** mindestens ein Farbstoff gemäß wenigstens einem der Ansprüche 1 bis 4 mit mindestens einem Kunststoff, vorzugsweise in Granulatform, vermischt oder vermahlen wird und das so erhaltene Gemisch geschmolzen und homogenisiert wird.

10. Verfahren zum Massefärben von Kunststoffen, **dadurch gekennzeichnet, dass** mindestens ein Farbstoff gemäß wenigstens einem der Ansprüche 1 bis 4 mit mindestens einem geschmolzenen Kunststoff vermischt wird und diese Mischung anschließend homogenisiert wird.

11. Verfahren zum Massefärben von Polymethylmethacrylat (PMMA) **dadurch gekennzeichnet, dass** mindestens ein Farbstoff gemäß wenigstens einem der Ansprüche 1 bis 4 mit mindestens einem Methacrylsäure-methylestermonomer vermischt oder darin gelöst wird und diese Mischung oder Lösung dann in Gegenwart mindestens eines Polymerisationskatalysators polymerisiert wird.

12. Kunststoffzusammensetzung, insbesondere Polystyrol, Polymethylmethacrylat, Polyethylenterephthalat oder Polycarbonat, **dadurch gekennzeichnet, dass** sie mindestens einen Farbstoff gemäß wenigstens einem der Ansprüche 1 bis 4 enthält.

13. Formteile, **dadurch gekennzeichnet, dass** sie mindestens eine Kunststoffzusammensetzung gemäß Anspruch 12 enthalten.

14. Verfahren zur Herstellung eines Farbstoffs gemäß Anspruch 1, **dadurch gekennzeichnet, dass** mindestens ein Aldehyd der Formel (II) 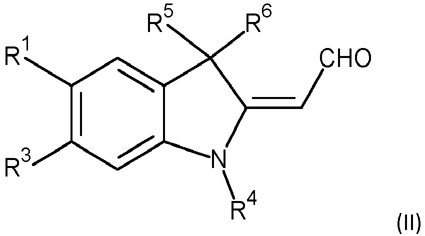 worin
R¹, R³, R⁴, R⁵ und R⁶ die in Anspruch 1 angegebenen Bedeutungen haben,
mit mindestens einer Verbindung der Formel (III) 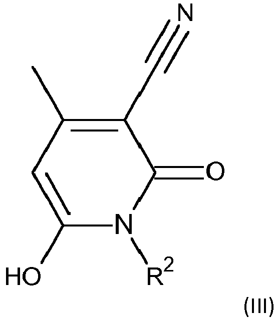 worin
R², R⁷ und R⁸ die in Anspruch 1 angegebenen Bedeutungen haben, umsetzt.
